# FASCICULE DE BREVET EUROPEEN

(11) **EP 1 818 038 B1**
(45) Date de publication et mention de la délivrance du brevet: **22.10.2008**
(21) Numéro de dépôt: 07356016.1
(22) Date de dépôt: 07.02.2007
(51) Int. Cl.: A61H 35/00, A47K 3/022, F21V 33/00, A61N 5/06, A47K 3/02, A47K 3/00

(54) **Appareil de bain de pieds**
Fußbadvorrichtung
Foot bath device

(30) Priorité: 10.02.2006 FR 0601193
(43) Date de publication de la demande: 15.08.2007
(73) Titulaire: SEB SA, 69130 Ecully (FR)
(72) Inventeur: Legrain, Marc, 01390 Civrieux en Dombes (FR)
(74) Mandataire: Kiehl, Hubert

(56) Documents cités:
- EP-A2- 1 256 303
- WO-A2-03/034883
- DE-A1- 10 312 687
- DE-A1- 19 747 980
- JP-A- 3 212 239
- JP-A- 2001 169 949
- US-A- 4 535 489

## Description

La présente invention est relative à un appareil adapté pour le traitement ou le bain de pieds, notamment du type apte à réaliser le lavage et également le massage des pieds et concerne plus particulièrement un dispositif d'éclairage pour un tel appareil.

Les appareils de bain pour les pieds sont utilisés en raison de l'effet décontractant procuré, dans un but hygiénique de nettoyage ainsi que pour l'amélioration de la circulation sanguine. Les appareils disposent la plupart du temps de fonctionnalités permettant un massage plantaire dans un but relaxant et parfois thérapeutique lorsque il est envisagé de stimuler les zones réflexes des pieds sur les points de réflexologie indiqués dans la médecine traditionnelle chinoise.

Un tel appareil pour le bain de pieds comporte généralement une cuve prévue pour recevoir une certaine quantité d'eau, comportant éventuellement des moyens de chauffage de l'eau, ainsi qu'un dispositif à entraînement électromécanique apte à produire des vibrations dans les parois de la cuve ou à envoyer des jets d'eau pulsés vers les pieds. Certains appareils plus perfectionnés comportent un dispositif à entraînement électromécanique supplémentaire agencé au centre de la cuve pour entraîner en un mouvement de rotation, de translation ou de vibration un ou plusieurs accessoires de massage disposés au dessus du niveau d'eau dans la cuve. Il a été constaté qu'un tel massage localisé de la plante des pieds contribue à améliorer la circulation sanguine et à stimuler le système nerveux périphérique, ce qui provoque une sensation de détente et de relaxation de la partie du corps traitée. Un tel mouvement appliqué aux pieds arrive, de plus, à dissiper les sensations de fatigue ressenties dans les pieds ou les jambes.

Le document US 4 497 313 décrit un tel appareil pour le bain de pieds comportant une cuve contenant de l'eau, la cuve étant mise en vibration par un dispositif situé en dessous, une pompe pour produire un jet d'eau en direction des pieds et, de surcroît, un dispositif de chauffage par rayonnement infrarouge ainsi qu'une lampe témoin. Le dispositif de chauffage et la lampe témoin sont situés à l'extérieur de la cuve et sont destinés à irradier les pieds et l'eau dans la cuve. Un tel chauffage à rayonnement infrarouge augmente le confort et la sensation de bien être de l'utilisateur lorsqu'il plonge ses pieds dans la cuve.

Toutefois, l'effet relaxant dépend des fonctionnalités de l'appareil bien évidemment mais aussi de la manière dont l'utilisateur vit le moment de relaxation, ce moment de relaxation peut également être amélioré par des effets favorables sur les autres sens par une action sur l'environnement immédiat de l'appareil.

On connaît par ailleurs, des baignoires lumineuses aptes à colorer l'eau du bain, mais où les dispositifs d'éclairage sont montés dans un boîtier recouvert d'une fenêtre transparente inséré dans la paroi de la baignoire pour obtenir une meilleure diffusion du faisceau lumineux dans le volume d'eau de la baignoire. Une telle solution nécessite l'utilisation de systèmes d'étanchéité qui sont souvent coûteux et requièrent une maintenance régulière.

On connaît également des documents EP 1 256 303, JP 3-212239, JP 2001-169949, DE 103 12 687, US 4 535 489 des baignoires comportant des dispositifs lumineux agencés derrière une paroi transparente. Un tel agencement nécessite toutefois l'aménagement d'un espace supplémentaire autour de la cuve de la baignoire, nécessite également l'agencement de plusieurs sources lumineuses uniformément réparties le long des parois avec, pour conséquence directe, l'augmentation du gabarit et celle du coût de réalisation de l'ensemble.

Le même problème se pose avec la cuvette de lavage des cheveux du document WO 03/034883 comportant un récipient interne réalisé en un matériau perméable à la lumière et des lampes agencées sur le pourtour externe du récipient. Ce document présente, en outre, le désavantage de ne pas permettre à l'utilisateur de visualiser la lumière émise, car elle jaillit derrière la tête de l'utilisateur.

Les appareils de bain de pieds existants, eux, ne présentent pas à ce jour de dispositifs permettant de placer l'utilisateur dans une ambiance douce, favorable à la relaxation.

L'invention a pour objectif de répondre à ce besoin de conditionnement de l'utilisateur dans une atmosphère propice à la relaxation en proposant un appareil de bain de pieds comportant un dispositif d'éclairage apte à délivrer un effet lumineux dans l'eau du bain de manière simple, fiable et économique.

Un autre but de l'invention est un appareil de bain de pieds comportant un dispositif d'éclairage compact, apte à être contenu dans son boîtier, tout en étant de fonctionnement sécuritaire pour l'utilisateur.

Un autre but de l'invention est un appareil de bain de pieds comportant un dispositif d'éclairage apte à varier la couleur de l'eau du bain et d'en assurer la diffusion uniforme dans l'eau du bain, ceci de manière simple, fiable et économique.

Un autre but de l'invention est un appareil de bain de pieds comportant un dispositif d'éclairage apte à varier la couleur de l'eau du bain de manière automatique, sans intervention de l'utilisateur à chaque changement de couleur.

Ces buts sont atteints avec un appareil de bain de pieds comportant une cuve pouvant contenir un certain volume d'eau et recevoir les pieds de l'utilisateur, ladite cuve étant supportée par un boîtier extérieur, du fait qu'il comprend un dispositif d'éclairage agencé dans l'espace fermé compris entre le boîtier extérieur et la cuve, sensiblement au centre de l'appareil, à l'intérieur d'une protubérance de la cuve, apte à émettre depuis son emplacement un rayonnement lumineux qui est visible dans un espace intérieur de la cuve à travers les parois de celle-ci.

L'appareil de bain de pieds de l'invention comprend donc un dispositif d'éclairage comportant au moins une source lumineuse agencée dans l'espace fermé qui sépare la cuve de son boîtier, donc dans une zone hors eau. Le rayonnement émis alors par un dispositif d'éclairage unique agencé derrière la cuve est apte à traverser les parois de la cuve et colorer le volume d'eau qu'elle contient. Ceci est dû à la puissance et à la longueur d'onde du rayonnement émis qui est choisi de manière à pouvoir traverser des parois en matière plastique opaques de faible épaisseur (par exemple inférieure à 10 mm), ou au fait que la cuve est réalisée en un matériau transparent ou translucide sur au moins une partie de sa surface permettant de rendre visible même un rayonnement de faible puissance.

La protubérance présente généralement une forme allongée, s'étendant selon l'axe médian de la cuve, sur presque toute la longueur de celle-ci. La protubérance définit ainsi avec le volume intérieur de la cuve deux emplacements pour les pieds de l'utilisateur. La hauteur de la protubérance dépasse de préférence le niveau de l'eau dans la cuve. Le dispositif d'éclairage est agencé au centre de l'appareil, donc de la protubérance, de manière à pouvoir éclairer, à partir d'un seul emplacement au centre, la totalité du volume d'eau de la cuve.

De par son agencement hors eau, le dispositif d'éclairage de l'appareil fournit la possibilité d'illuminer l'eau du bain, à partir d'un seul puits de lumière et sans nécessiter de système d'étanchéité au niveau de l'agencement de la source lumineuse, les parois de la cuve étant continues. Ceci simplifie la construction de l'appareil, le rend plus économique et fiable dans le temps, tout en étant d'une utilisation très agréable.

Avantageusement, la protubérance de la cuve comporte au moins un hublot en un matériau transparent ou translucide surmoulé avec les parois de la cuve et il est situé de préférence sous le niveau d'eau dans la cuve.

Cette solution permet de diffuser la lumière dans l'eau du bain, sans avoir à réaliser la cuve en un matériau transparent, donc en masquant les composants techniques de l'appareil pour ne laisser visible que la lumière qui jaillit alors par le ou les hublot(s).

On aurait pu, certes, envisager l'utilisation d'un même matériau opaque ou semi transparent et varier ensuite l'épaisseur de la paroi, par exemple en la rendant plus faible à l'emplacement choisi pour le passage du rayonnement lumineux. On préfère toutefois réaliser la cuve par moulage de deux matériaux différents, par exemple du polycarbonate pour réaliser le hublot et par exemple du polypropylène pour le corps de la cuve afin de garantir la continuité des parois et de permettre une meilleure visibilité du rayonnement émis, sans avoir à utiliser des joints ou autres systèmes d'étanchéité. Le hublot ainsi réalisé est avantageusement agencé sous le niveau de l'eau afin de garantir une meilleure diffusion de la lumière dans le volume d'eau contenu par la cuve.

Avantageusement, la protubérance comprend deux hublots transparents placés de chaque côté de la protubérance, de préférence à la base de la paroi de fond de la cuve, au même niveau que la source de lumière du dispositif d'éclairage. Suite à des tests effectués en laboratoire, il a été établi que deux hublots latéraux, ayant chacun une surface comprise de préférence entre 1cm² et 10cm² diffusaient suffisamment bien la lumière à partir d'une source située au centre de l'appareil en direction des pieds et dans la totalité du volume d'eau de la cuve d'un appareil pour le bain de pieds.

De préférence, le dispositif d'éclairage comprend au moins un système permettant le changement automatique de couleur du rayonnement lumineux émis.

Ceci permet de créer une ambiance agréable lors du bain de pieds au moyen de couleurs choisies et changeantes générant une atmosphère douce, amplifiant la sensation de relaxation de l'utilisateur. Un tel système peut, par exemple, utiliser au moins une diode luminescente multi couleurs qui intègre une puce et est ainsi apte, lorsque pilotée par un circuit électronique de l'appareil, à émettre différentes longueurs d'onde. La variation de couleur se fait de manière automatique, en étant de préférence choisie ou programmée à l'avance par l'utilisateur.

Dans un premier mode de réalisation de l'invention, le système permettant le changement de couleur comporte une source lumineuse unique associée à des filtres.

Ceci représente une solution simple et économique pour obtenir une variation de la couleur de l'eau avec une seule source lumineuse, par exemple une lampe émettant de la lumière blanche.

Dans une première variante de ce mode de réalisation, les filtres sont portés par un cylindre coloré entourant la source lumineuse, ledit cylindre coloré étant actionné en un mouvement de rotation au moyen d'un moto-réducteur.

Ainsi, en plaçant la source lumineuse selon l'axe du cylindre, à l'intérieur de celui-ci que l'on actionne en rotation par un moto-réducteur, on obtient une bonne diffusion, uniforme et cyclique, de lumière colorée dans le volume d'eau de la cuve.

Dans une deuxième variante de réalisation du premier mode, les filtres sont portés par un disque coloré situé au-dessus de la source lumineuse, ledit disque coloré étant actionné en un mouvement de rotation au moyen d'un moto-réducteur.

Cette variante présente, elle, l'avantage d'une plus grande compacité, la protubérance de la cuve pouvant facilement recevoir un mécanisme d'actionnement des dispositifs de massage à côté d'un tel disque coloré fin.

Avantageusement, la source lumineuse, les filtres et le moto-réducteur sont placés sensiblement au centre de la cuve à l'intérieur de la protubérance de celle-ci.

Cet agencement s'avère très judicieux, car il n'y a nullement besoin d'augmenter le gabarit de l'appareil, les appareils de bain de pied disposant généralement d'un repose pied au centre de la cuve. En plaçant la source lumineuse, le filtre et le moto-réducteur au centre de la cuve, la lumière peut alors être uniformément diffusée, sans perte d'intensité lumineuse dans toute la cuve.

Dans un deuxième mode de réalisation, le dispositif d'éclairage comporte plusieurs sources lumineuses de différentes couleurs.

Ces sources lumineuses sont placées au centre de la cuve et sont visibles à travers les parois ou les hublots de la protubérance centrale, offrant en même temps une réalisation économique et plus de flexibilité à l'utilisateur qui peut alors choisir soit de commander indépendamment chaque source lumineuse, selon ses propres paramètres, soit de commander de manière automatique plusieurs sources lumineuses en même temps pour obtenir une combinaison de teintes et nuances.

Avantageusement, les sources lumineuses sont commandées par un circuit électronique gérant les variations des couleurs et la synchronisation des séquences colorées.

Les teintes et les séquences de défilement des couleurs peuvent être choisies selon les préconisations de la luminothérapie ou de la chromothérapie de manière à amener un effet psychique complémentaire au bien être procuré par les fonctions traditionnellement disposées sur l'appareil. La synchronisation peut être réalisée selon des paramètres préenregistrés (vitesse, période) ou des paramètres choisis et réglés par l'utilisateur. Dans une variante, on pourrait utiliser un lecteur de musique, par exemple un lecteur MP3, intégré à l'appareil pour diffuser de la musique (moyennant un casque ou des hauts parleurs) et, en même temps, faire varier la couleur en fonction de cette musique, choisie de préférence, douce et relaxante.

Dans un exemple de réalisation de l'invention, le système permettant le changement de couleur du rayonnement lumineux émis par le dispositif d'éclairage est commandé par un circuit électronique gérant les variations des couleurs en fonction de la température de l'eau dans la cuve.

Dans cette réalisation, l'appareil comprend un capteur de température qui mesure la température de l'eau contenue par la cuve, le capteur étant relié au circuit électronique de l'appareil. Ainsi, le circuit électronique peut commander une émission de couleur qui est en fonction de la température du bain, couleur choisie selon un code de couleurs préétabli, par exemple bleu si l'eau est froide, orange ou rouge si le bain est bien chaud. Ceci permet donc d'informer l'utilisateur de la température du bain, par exemple, avant de commencer le traitement. Par ailleurs, une fois la température de l'eau mesurée et le traitement commencé, le circuit électronique peut démarrer un nouveau cycle de variation de couleurs en fonction d'autres paramètres préréglés ou choisis par l'utilisateur.

Avantageusement, le rayonnement émis par le dispositif d'éclairage est transporté vers au moins un hublot au moyen d'un guide d'onde ou d'une fibre optique.

Ceci permet de conduire sans pertes au plus près de la paroi de la cuve, mais derrière la cuve, le rayonnement émis par un dispositif lumineux agencé à distance des parois de la cuve, la variation de lumière correspondant à la lumière ayant préalablement traversé un filtre en mouvement ou à la lumière issue d'une modulation programmée d'un ensemble de sources lumineuses de différentes couleurs.

De préférence, ledit dispositif d'éclairage comporte un réflecteur de lumière favorisant la diffusion de lumière à travers au moins un hublot transparent ou translucide.

Un tel réflecteur permet alors de renvoyer la lumière en direction du hublot et éventuellement la concentrer, lorsqu'il est par exemple parabolique, pour une meilleure diffusion de la lumière dans l'eau de la cuve.

D'autres caractéristiques et avantages de l'invention se dégageront de la description qui va suivre en regard des dessins annexés qui ne sont donnés qu'à titre d'exemples non limitatifs :
- la figure 1 est une vue en perspective et de dessus d'un appareil pour bain de pieds comportant l'invention selon une première variante d'un premier mode de réalisation;
- la figure 2 est une vue en coupe de l'appareil pour bain de pieds illustré sur la figure 1;
- la figure 3 est une vue en perspective et de dessus d'un appareil pour bain de pieds comportant l'invention selon une deuxième variante du premier mode de réalisation;
- la figure 4 est une vue en coupe de l'appareil pour bain de pieds illustré sur la figure 3;
- la figure 5 est une vue en perspective et de dessus d'un appareil pour bain de pieds comportant l'invention selon un deuxième mode de réalisation;
- la figure 6 est une vue en coupe de l'appareil pour bain de pieds illustré sur la figure 5.

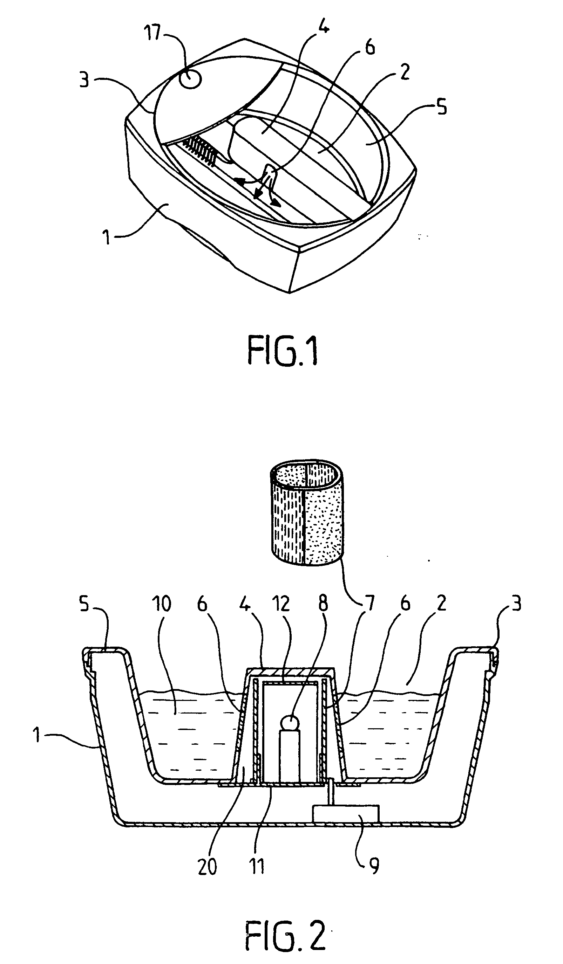

La figure 1 montre un appareil pour bain de pieds comportant un boîtier extérieur 1 et une cuve 3 présentant un espace intérieur 2 qui est étanche et est destiné à recevoir de l'eau 10 ainsi qu'à permettre à l'utilisateur de placer ses pieds. Une protubérance 4 de forme allongée placée au centre de l'espace intérieur 2 de l'appareil, selon un axe médian de la cuve, sépare l'espace intérieur 2 en deux compartiments qui communiquent entre eux, un compartiment étant prévu pour chaque pied. La protubérance 4 forme appui pour le pied et peut, dans une variante, supporter en sa partie supérieure des accessoires de massage ou de traitement des pieds entraînés en mouvement par un moteur électrique et un train d'engrenages agencés à l'intérieur de la protubérance 4 (non représentés sur les dessins). L'appareil est par ailleurs muni de moyens de chauffage et d'un réglage de température 17 accessible par l'utilisateur.

Selon l'invention, l'espace fermé compris entre la cuve 3 et le boîtier extérieur 1, à l'intérieur de la protubérance 4, renferme un dispositif d'éclairage 20 comportant au moins une source de lumière de manière à délivrer dans l'espace intérieur 2 une lumière, de préférence douce et agréable. Le dispositif d'éclairage 20, ses composants et ses connexions électriques sont agencés donc hors eau, dans un espace étanche, en étant derrière les parois de la cuve. La cuve est généralement obtenue par moulage en une matière plastique, par exemple du polypropylène d'une épaisseur comprise entre 3 et 8 mm.

De préférence selon l'invention, l'espace intérieur 2 de l'appareil comporte une paroi étanche 5 pourvue d'au moins un hublot 6 transparent ou translucide permettant la diffusion de la lumière dans l'espace intérieur 2, notamment à travers le liquide du bain. Les hublots 6 en une matière transparente ou translucide, par exemple du polycarbonate, sont prévus dans les parois latérales de la protubérance 4, au niveau de l'espace intérieur 2 et sous le niveau d'eau dans la cuve 3. En plaçant les hublots de chaque côté de la protubérance 4 séparant l'espace intérieur en deux compartiments, sur les parois latérales dudit boîtier on arrive à illuminer toute l'eau du bain par les côtés. Dans une variante, un hublot supplémentaire pourrait être prévu sur la paroi frontale de la protubérance 4 pour illuminer également le volume d'eau situé à l'avant des pieds,

Dans une autre variante, on peut imaginer que la cuve est entièrement réalisée en un matériau transparent ou translucide.

Il est également concevable, sans sortir du cadre de l'invention, de déporter la source lumineuse sur un bord ou sur le dessus de l'appareil et d'amener la variation de lumière vers différents hublots, disposés en divers points de la paroi étanche 5, au moyen d'un réseau de fibres optiques ou de guides d'onde.

Avantageusement selon l'invention le dispositif d'éclairage 20 comporte un système permettant le changement automatique de couleur du rayonnement émis, notamment avec des teintes changeantes et une succession de nuances susceptibles d'améliorer l'effet relaxant produit par l'appareil. Selon l'invention les variations de lumière peuvent être engendrées de différentes manières, les exemples de réalisation décrivent des moyens économiques et fiables utilisables avec de nombreux modèles d'appareils.

Ainsi, la variation de lumière peut être avantageusement obtenue par l'emploi d'une source lumineuse unique associée à des filtres colorés en mouvement ou par plusieurs sources lumineuses de couleurs différentes modulées en intensité de manière à fournir des changements de teintes. Toutefois, sans sortir du cadre de l'invention, il est envisageable d'employer des dispositifs plus sophistiqués tels que des filtres avec des cristaux liquides modifiables sous l'action d'un champ électrique, et, de manière générale, tout dispositif susceptible de produire des variations de couleur sur le parcours d'un faisceau lumineux peut être utilisé. Ainsi, on pourrait envisager l'utilisation d'une lampe électroluminescente à base de phosphore déposée sur un film flexible et qui serait ensuite collée aux parois de la protubérance 4 de la cuve 3, dans l'espace fermé défini entre celle-ci et le boîtier extérieur 1.

Une caractéristique importante de l'invention, favorable pour la fabrication économique du dispositif, est que les moyens permettant le changement de couleur de l'espace intérieur 2 sont situés dans le corps de l'appareil, dans une zone hors eau, ce qui évite l'emploi d'éléments complexes et coûteux. Les hublots transparents ou translucides surmoulés avec les parois latérales de la protubérance 4 ne posent pas de problème d'installation et peuvent être conçus parfaitement étanches.

Selon un premier mode de réalisation illustré sur les figures 1 à 4, le système permettant le changement de couleur de l'espace intérieur 2 de l'appareil pour bain de pieds comporte une source lumineuse unique associée à des filtres.

Dans une première variante de ce premier mode de réalisation qui est visible aux figures 1 et 2, les filtres colorés sont portés par un cylindre coloré 7 entourant la source lumineuse 8. Ledit cylindre coloré 7 est actionné en un mouvement de rotation autour de la source lumineuse 8 au moyen d'un moto-réducteur 9 placé au fond de l'appareil. Le cylindre coloré est conçu avec un agencement de plastiques colorés soudés ou assemblés sur le pourtour du cylindre, l'enchaînement et l'organisation des filtres sur la périphérie du cylindre produisant différents effets colorés et des transitions plus ou moins prononcées entre les couleurs, il existe de fait de nombreuses variations possibles sans sortir du cadre de l'invention. Les effets colorés peuvent également être réglés au moyen de la vitesse de rotation du filtre, soit donc par l'intermédiaire du moto-réducteur 9.

La source lumineuse 8 et le cylindre coloré 7 sont placés sensiblement au centre de l'appareil pour bain de pieds, à l'intérieur de la protubérance 4. Préférentiellement l'appareil comporte deux hublots 6 placés sur les parois latérales de la protubérance 4. La figure 2 illustre la position de ces hublots 6 et plus particulièrement le fait qu'ils sont prévus dans la zone immergée, soit sous le niveau de l'eau 10 qui remplit l'espace intérieur de l'appareil.

La figure 2 montre en coupe la protubérance 4 contenant la source lumineuse 8 sensiblement en son centre, positionnée de manière à diffuser la lumière au travers des deux hublots. Avantageusement selon l'invention les éléments principaux, à l'exception du moto-réducteur 9, sont supportés par une plaque 11 ou un boîtier inférieur servant de support. Un réflecteur 12 est agencé dans la partie supérieure de la protubérance 4 afin que la lumière soit renvoyée vers les hublots 6. Ce réflecteur pouvant être un miroir plan tel que cela est illustré sur la figure 2 ou un dispositif quelconque de renvoi, ou de focalisation, de la lumière.

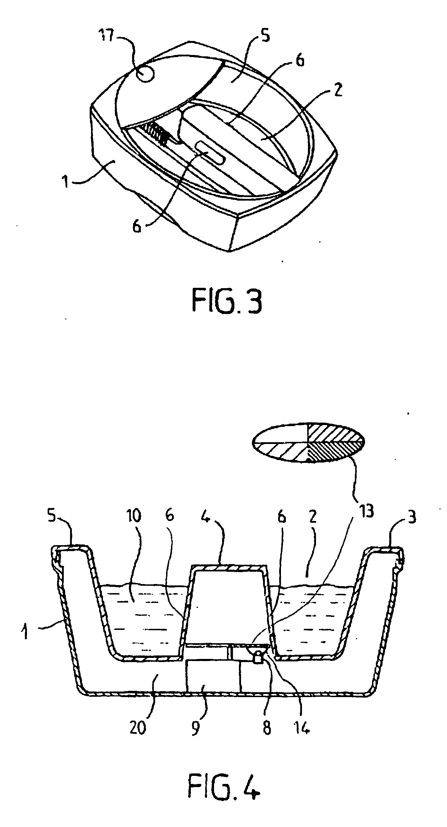

Selon une deuxième variante de réalisation illustrée sur les figures 3 et 4, les filtres sont portés par un disque coloré 13 placé au dessus de la source lumineuse, ledit disque coloré 13 étant actionné en un mouvement de rotation au moyen d'un moto-réducteur 9 de manière à faire défiler entre la source lumineuse 8 et le hublot 6 différentes plages de couleur.

Avantageusement selon cet exemple de réalisation la source lumineuse 8, le disque coloré 13, un réflecteur 14 et le moto-réducteur 9 sont placés sensiblement au centre de la protubérance 4, de manière à diffuser la lumière latéralement vers les pieds de l'utilisateur au travers deux hublots 6 placés de chaque côté de la protubérance 4 dans la base de la paroi étanche 5. Le réflecteur de lumière 14 est prévu immédiatement sous la source lumineuse de manière à orienter le faisceau coloré vers le haut, c'est à dire vers les hublots et les pieds de l'utilisateur.

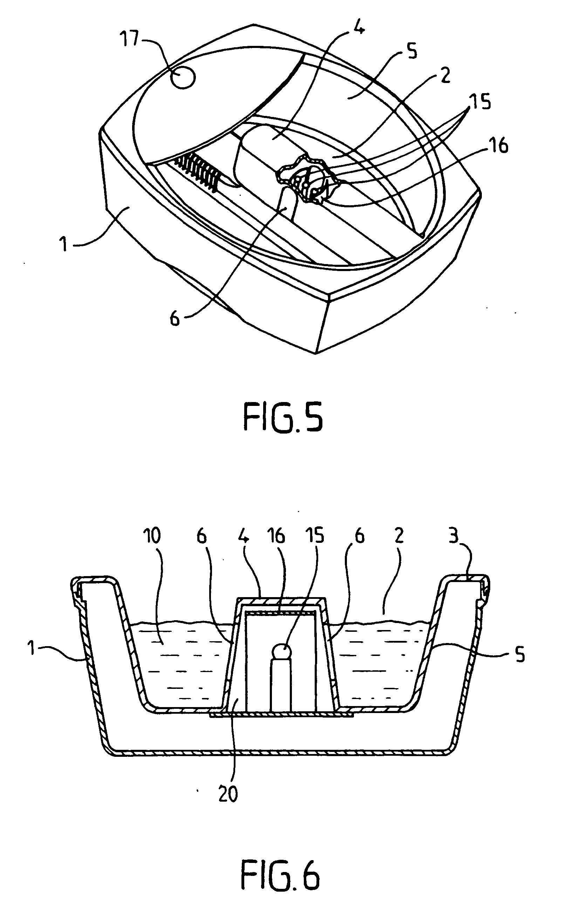

Selon un deuxième mode de réalisation de l'invention illustré dans les figures 5 et 6 le dispositif d'éclairage 20 comporte plusieurs sources lumineuses 15 de différentes couleurs. On peut ainsi prévoir d'associer trois sources lumineuses, par exemple des diodes électroluminescentes de couleurs jaune, rouge et bleue et un dispositif de contrôle pour commander soit une seule diode à la fois, soit au moins deux diodes simultanément de manière à réaliser par combinaison, en jouant sur l'intensité de chaque source, une palette variée de couleurs. Comme dans les autres exemples de réalisation il est avantageux de placer un réflecteur 16 de lumière pour orienter le faisceau vers les hublots. Dans ce mode de réalisation également, l'ensemble des sources lumineuses 15 sera préférentiellement placé sensiblement au centre de l'appareil pour bain de pieds, soit à l'intérieur de la protubérance 4.

Avantageusement, dans ce mode de réalisation, les sources lumineuses sont commandées par un circuit électronique gérant les variations des couleurs et la synchronisation des séquences. Il peut aussi être envisagé d'asservir la séquence de coloration avec la température du bain, par exemple une variation des nuances dans le bleu si le bain est froid ou dans le rouge et l'orangé si le bain est chaud. Des cycles colorés programmables par l'utilisateur lui-même ou la sélection de séquences préprogrammées peuvent aussi être envisagés sans sortir du cadre de l'invention.

Dans ce mode de réalisation, comme dans les précédents, la variation de lumière peut être transportée vers au moins un hublot au moyen d'un guide d'onde ou d'un réseau de fibres optiques si la ou les sources lumineuses sont déportées sur la périphérie ou sur le dessus de l'appareil.

Il a été décrit précédemment différents exemples de réalisation, plusieurs autres variantes sont possibles quant à la localisation des moyens d'éclairage et aux dispositifs permettant de faire varier la couleur à l'intérieur du liquide dans l'appareil pour bain de pieds, sans sortir du cadre de l'invention.

De même la taille, le nombre et la localisation des hublots peuvent varier d'un appareil à un autre, et le dispositif de variation de couleur peut se trouver associé à n'importe laquelle des fonctionnalités habituellement rencontrées sur ces appareils.

L'appareil de l'invention peut également comporter des moyens de traitement ou de massage des pieds agencés en la partie supérieure d'une protubérance centrale de la cuve ou au fond de la cuve.

## Revendications

1. Appareil de bain de pieds comportant une cuve (3) pouvant contenir un certain volume d'eau et recevoir les pieds de l'utilisateur, ladite cuve (3) étant supportée par un boîtier extérieur (1), **caractérisé en ce qu'**il comprend un dispositif d'éclairage (20) agencé dans l'espace fermé compris entre le boîtier extérieur (1) et la cuve (3), sensiblement au centre de l'appareil, à l'intérieur d'une protubérance (4) de la cuve (3), apte à émettre depuis son emplacement un rayonnement lumineux qui est visible dans un espace intérieur (2) de la cuve (3) à travers les parois de celle-ci.

2. Appareil selon la revendication 1, **caractérisé en ce que** la protubérance (4) de la cuve (3) comporte au moins un hublot (6) en un matériau transparent ou translucide surmoulé avec les parois de la cuve et qu'il est situé de préférence sous le niveau d'eau dans la cuve (3).

3. Appareil selon l'une des revendications 1 ou 2, **caractérisé en ce que** le dispositif d'éclairage (20) comprend au moins un système permettant le changement automatique de couleur du rayonnement lumineux émis.

4. Appareil selon la revendication 3, **caractérisé en ce que** le système permettant le changement de couleur comporte une source lumineuse (8) unique associée à des filtres.

5. Appareil selon la revendication 4, **caractérisé en ce que** les filtres sont portés par un cylindre coloré (7) entourant la source lumineuse (8), ledit cylindre coloré (7) étant actionné en un mouvement de rotation au moyen d'un moto-réducteur (9).

6. Appareil selon la revendication 4, **caractérisé en ce que** les filtres sont portés par un disque coloré (13) situé au-dessus de la source lumineuse (8), ledit disque coloré (13) étant actionné en un mouvement de rotation au moyen d'un moto-réducteur (9).

7. Appareil selon l'une des revendications 5 ou 6, **caractérisé en ce que** la source lumineuse (8), les filtres et le moto-réducteur (9) sont placés sensiblement au centre de la cuve (3) à l'intérieur de la protubérance (4) de celle-ci.

8. Appareil selon l'une des revendications 1 à 3, **caractérisé en ce que** le dispositif d'éclairage (20) comporte plusieurs sources lumineuses (15) de différentes couleurs.

9. Appareil selon la revendication 8, **caractérisé en ce que** les sources lumineuses (15) sont commandées par un circuit électronique gérant les variations des couleurs et la synchronisation des séquences colorées.

10. Appareil selon la revendication 3, **caractérisé en ce que** le système permettant le changement de couleur du rayonnement lumineux émis par le dispositif d'éclairage (20) est commandé par un circuit électronique gérant les variations des couleurs en fonction de la température de l'eau dans la cuve (3).

11. Appareil selon l'une des revendications précédentes, **caractérisé en ce que** le rayonnement émis par le dispositif d'éclairage (20) est transporté vers au moins un hublot (6) au moyen d'un guide d'onde ou d'une fibre optique.

12. Appareil selon l'une des revendications précédentes, **caractérisé en ce que** ledit dispositif d'éclairage (20) comporte un réflecteur (12, 14, 16) de lumière favorisant la diffusion de lumière à travers au moins un hublot (6) transparent ou translucide.

## Claims

1. A footbath appliance comprising a basin (3) that can contain a certain volume of water and that can receive the user's feet, said basin (3) being supported by an outer housing (1), said footbath appliance being **characterized in that** it further comprises a lighting device (20) arranged in the closed space lying between the outer housing (1) and the basin (3), substantially at the center of the appliance, inside a protuberance (4) in the basin (3), and suitable for emitting, from its location, light radiation that is visible in an internal space (2) of the basin (3) through the walls thereof.

2. An appliance according to claim 1, **characterized in that** the protuberance (4) in the basin (3) is provided with at least one window (6) made of a transparent or translucent material that is embedded in the walls of the basin by molding and that is preferably situated under the water level in the basin (3).

3. An appliance according to claim 1 or claim 2, **characterized in that** the lighting device (20) comprises at least one system for automatically changing the color of the emitted light radiation.

4. An appliance according to claim 3, **characterized in that** the system for changing the color comprises a single light source (8) associated with filters.

5. An appliance according to claim 4, **characterized in that** the filters are carried by a colored cylinder (7) surrounding the light source (8), said colored cylinder (7) being actuated in rotation by means of a motor-and-gearbox unit (9).

6. An appliance according to claim 4, **characterized in that** the filters are carried by a colored disk (13) situated above the light source (8), said colored disk (13) being actuated in rotation by means of a motor-and-gearbox unit (9).

7. An appliance according to claim 5 or claim 6, **characterized in that** the light source (8), the filters and the motor-and-gearbox unit (9) are placed substantially at the center of the basin (3) inside the protuberance (4) therein.

8. An appliance according to any one of claims 1 to 3, **characterized in that** the lighting device (20) comprises a plurality of light sources (15) of different colors.

9. An appliance according to claim 8, **characterized in that** the light sources (15) are controlled by an electronic circuit managing the variations in the colors and the synchronization of the colored sequences.

10. An appliance according to claim 3, **characterized in that** the system making it possible to change the color of the light radiation emitted by the lighting device (20) is controlled by an electronic circuit managing the variations in colors as a function of the temperature of the water in the basin (3).

11. An appliance according to any preceding claim, **characterized in that** the radiation emitted by the lighting device (20) is conveyed towards at least one window (6) by means of a waveguide or of an optical fiber.

12. An appliance according to any preceding claim, **characterized in that** said lighting device (20) further comprises a light reflector (12, 14, 16) facilitating the diffusion of light through at least one transparent or translucent window (6).

## Patentansprüche

1. Fußbadvorrichtung mit einer Wanne (3), die ein bestimmtes Wasservolumen enthalten und die Füße des Benutzers aufnehmen kann, wobei die Wanne (3) von einem Außengehäuse (1) getragen ist, **dadurch gekennzeichnet, dass** sie eine Beleuchtungseinrichtung (20) umfasst, die im geschlossenen Raum zwischen dem Außengehäuse (1) und der Wanne (3), im Wesentlichen in der Mitte der Vorrichtung, innerhalb einer Ausbauchung (4) der Wanne (3) angeordnet ist und ausgehend von ihrem Standort eine Lichtstrahlung aussenden kann, die in einem Raum innerhalb (2) der Wanne (3) durch deren Wände hindurch sichtbar ist.

2. Vorrichtung nach Anspruch 1, **dadurch gekennzeichnet, dass** die Ausbauchung (4) der Wanne (3) mindestens ein Sichtfenster (6) aus einem durchsichtigen oder durchscheinenden Material aufweist, das mit den Wänden der Wanne überformt ist, und dass es sich vorzugsweise unter dem Wasserspiegel in der Wanne (3) befindet.

3. Vorrichtung nach einem der Ansprüche 1 oder 2, **dadurch gekennzeichnet, dass** die Beleuchtungseinrichtung (20) mindestens ein System aufweist, das die automatische Farbänderung der ausgesendeten Lichtstrahlung ermöglicht.

4. Vorrichtung nach Anspruch 3, **dadurch gekennzeichnet, dass** das System, das die Farbänderung ermöglicht, eine einzige, Filtern zugeordnete Lichtquelle (8) aufweist.

5. Vorrichtung nach Anspruch 4, **dadurch gekennzeichnet, dass** die Filter von einem farbigen Zylinder (7) getragen sind, der die Lichtquelle (8) umgibt, wobei der farbige Zylinder (7) mittels eines Getriebemotors (9) in eine Drehbewegung versetzt wird.

6. Vorrichtung nach Anspruch 4, **dadurch gekennzeichnet, dass** die Filter von einer farbigen Scheibe (13) getragen sind, die oberhalb der Lichtquelle (8) angeordnet ist, wobei die farbige Scheibe (13) mittels eines Getriebemotors (9) in eine Drehbewegung versetzt wird.

7. Vorrichtung nach einem der Ansprüche 5 oder 6, **dadurch gekennzeichnet, dass** die Lichtquelle (8), die Filter und der Getriebemotor (9) im Wesentlichen in der Mitte der Wanne (3) innerhalb deren Ausbauchung (4) aufgestellt sind.

8. Vorrichtung nach einem der Ansprüche 1 bis 3, **dadurch gekennzeichnet, dass** die Beleuchtungseinrichtung (20) mehrere Lichtquellen (15) in verschiedenen Farben aufweist.

9. Vorrichtung nach Anspruch 8, **dadurch gekennzeichnet, dass** die Lichtquellen (15) von einer elektronischen Schaltung gesteuert sind, die die Farbvariationen und die Synchronisierung der farbigen Sequenzen verwaltet.

10. Vorrichtung nach Anspruch 3, **dadurch gekennzeichnet, dass** das System, das die Änderung der Farbe der ausgesendeten Lichtstrahlung durch die Beleuchtungseinrichtung (20) ermöglicht, von einer elektronischen Schaltung gesteuert ist, die die Farbvariationen in Abhängigkeit von der Temperatur des Wassers in der Wanne (3) verwaltet.

11. Vorrichtung nach einem der vorhergehenden Ansprüche, **dadurch gekennzeichnet, dass** die von der Beleuchtungseinrichtung (20) ausgesendete Strahlung zu mindestens einem Sichtfenster (6) mittels eines Wellenleiters oder einer Lichtfaser geleitet wird.

12. Vorrichtung nach einem der vorhergehenden Ansprüche, **dadurch gekennzeichnet, dass** die Beleuchtungseinrichtung (20) einen Lichtreflektor (12, 14, 16) aufweist, der die Verbreitung von Licht durch mindestens ein durchsichtiges oder durchscheinendes Sichtfenster (6) begünstigt.
